# EUROPEAN PATENT APPLICATION

(11) **EP 2 386 251 A1**
(43) Date of publication of application: **16.11.2011**
(21) Application number: 11163624.7
(22) Date of filing: 26.04.2011
(51) Int. Cl.: A61B 8/14, G01S 7/52, G01S 15/89, G06T 19/00

(54) **Providing at least one slice image with additional information in an ultrasound system**

(30) Priority: 13.05.2010 KR 20100044770
(71) Applicant: Samsung Medison Co., Ltd., Nam-myun Hongchun-gun Kangwon do 250-875 (KR)
(72) Inventor: Kim, Sung Yoon, 135-851 Seoul (KR)
(74) Representative: Schmid, Wolfgang

(57) **Abstract**

Embodiments for providing at least one slice image with additional information are disclosed. In one embodiment, by way of non-limiting example, an ultrasound system (100) comprises: an ultrasound data acquisition unit (110) configured to acquire ultrasound data of a living body; a user input unit (120) configured to receive input information for setting at least three points (P1,P2,P3,P4) on a three-dimensional ultrasound image (UI) from a user; and a processing unit (130) being configured to form the three-dimensional ultrasound image (UI) based on the volume data, set the at least three points (P1,P2,P3,P4) on the three-dimensional ultrasound image (UI) based on the input information, set at least one slice (S1,S2,S3,S4) on the three-dimensional ultrasound image (UI) based on the at least three points (P1,P2,P3,P4) and form at least one slice image corresponding to the at least one slice (S1,S2,S3,S4) and additional information corresponding to positions of the at least three points (P1,P2,P3,P4) based on the volume data and the input information.

## Description

### CROSS-REFERENCE TO RELATED APPLICATIONS

The present application claims priority from Korean Patent Application No. 10-2010-0044770 filed on May 13, 2010.

### TECHNICAL FIELD

The present disclosure generally relates to ultrasound systems, and more particularly to providing at least one slice image with additional information in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two-dimensional (2D) or three-dimensional (3D) ultrasound images of internal features of an object (e.g., human organs).

The ultrasound system may provide the 3D ultrasound image including clinical information such as spatial information and anatomical figures of the target object, which cannot be provided by the 2D ultrasound image. The ultrasound system may transmit ultrasound signals into a target object and receive ultrasound echo signals reflected from the target object. The ultrasound system may further form volume data based on the ultrasound echo signals. The ultrasound system may further render the volume data to thereby form the 3D ultrasound image.

However, to find a region of interest from the 3D ultrasound image, a user may need to rotate or move the 3D ultrasound image. Also, it may be difficult to provide at least one slice image including a plurality of regions of interest from the 3D ultrasound image.

### SUMMARY

Embodiments for providing a plurality of slice images in an ultrasound system are disclosed herein. In one embodiment, by way of non-limiting example, an ultrasound system comprises: an ultrasound data acquisition unit configured to transmit and receive ultrasound signals to and from a living body to output ultrasound data; a user input unit configured to receive input information for setting at least three points on a three-dimensional ultrasound image from a user; and a processing unit in communication with the ultrasound data acquisition unit and the user input unit, the processing unit being configured to form volume data based on the ultrasound data, render the volume data to form the three-dimensional ultrasound image, set the at least three points on the three-dimensional ultrasound image based on the input information, set at least one slice on the three-dimensional ultrasound image based on the at least three points and form at least one slice image corresponding to the at least one slice and additional information corresponding to positions of the at least three points based on the volume data and the input information.

In another embodiment, there is provided a method of providing slice images with additional information, comprising: a) transmitting and receiving ultrasound signals to and from a living body to output ultrasound data; b) forming volume data based on the ultrasound data; c) rendering the volume data to form the three-dimensional ultrasound image; d) receiving input information for setting at least three points on the three-dimensional ultrasound image from a user; e) setting the at least three points on the three-dimensional ultrasound image based on the input information; f) setting at least one slice on the three-dimensional ultrasound image based on the at least three points; and g) forming at least one slice image corresponding to the at least one slice and additional information corresponding to positions of the at least three points.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a schematic diagram showing an example of acquiring ultrasound data corresponding to a plurality of frames.
FIG. 4 is a flow chart showing a process of forming at least one slice image based on at least three points set on a 3D ultrasound image.
FIG. 5 is a schematic diagram showing an example of volume data.
FIG. 6 is a schematic diagram showing an example of points set on the 3D ultrasound image.
FIG. 7 is a schematic diagram showing an example of slices set on the 3D ultrasound image.
FIG. 8 is a schematic diagram showing an example of additional information.
FIG. 9 is a schematic diagram showing an example of the additional information.

### DETAILED DESCRIPTION

A detailed description may be provided with reference to the accompanying drawings. One of ordinary skill in the art may realize that the following description is illustrative only and is not in any way limiting.

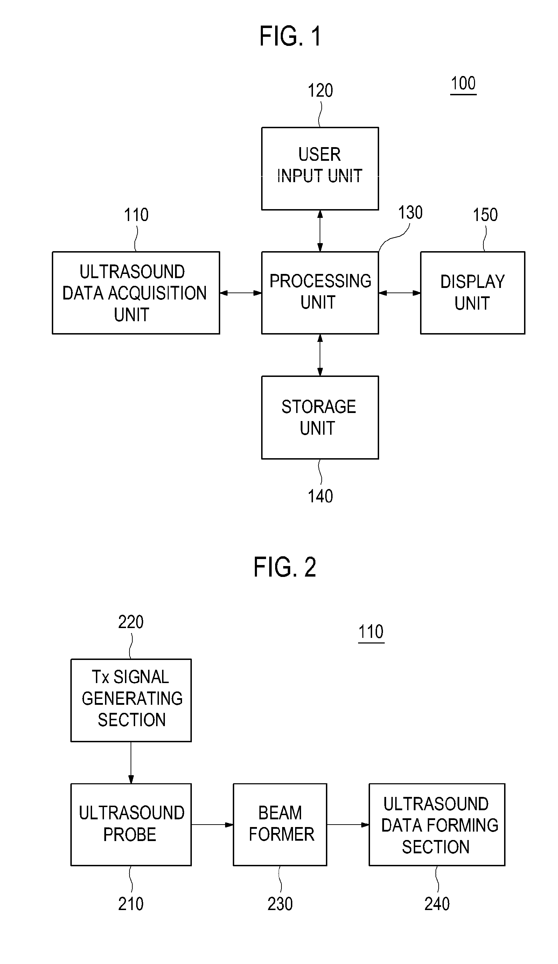

Referring to FIG. 1, an ultrasound system 100 in accordance with an illustrative embodiment is shown. As depicted therein, the ultrasound system 100 includes an ultrasound data acquisition unit 110. The ultrasound data acquisition unit 110 is configured to transmit and receive ultrasound signals to and from a living body, and output ultrasound data. The living body includes a plurality of target objects (e.g., blood vessels, a heart, etc.).

FIG. 2 is a block diagram showing an illustrative embodiment of the ultrasound data acquisition unit 110. Referring to FIG. 2, the ultrasound data acquisition unit 110 includes an ultrasound probe 210, a transmit (Tx) signal generating section 220, a beam former 230 and an ultrasound data forming section 240.

The ultrasound probe 210 includes a plurality of elements (not shown) for reciprocally converting between ultrasound signals and electrical signals. The ultrasound probe 210 is configured to transmit ultrasound signals to the living body. The ultrasound probe 210 further receives ultrasound signals (i.e., ultrasound echo signals) from the living body and output the received signals. The received signals are analog signals. The ultrasound probe 210 includes a three-dimensional (3D) mechanical probe or a two-dimensional (2D) array probe. However, it should be noted herein that the ultrasound probe 210 may not be limited thereto.

The Tx signal generating section 220 is configured to control the transmission of the ultrasound signals. The Tx signal generating section 220 further generates electrical signals ("Tx signals") in consideration of the elements and focal points. Thus, the ultrasound probe 210 converts the Tx signals provided from the Tx signal generating section 220 into the ultrasound signals, transmit the ultrasound signals to the living body and receive the ultrasound echo signals from the living body to thereby output the received signals.

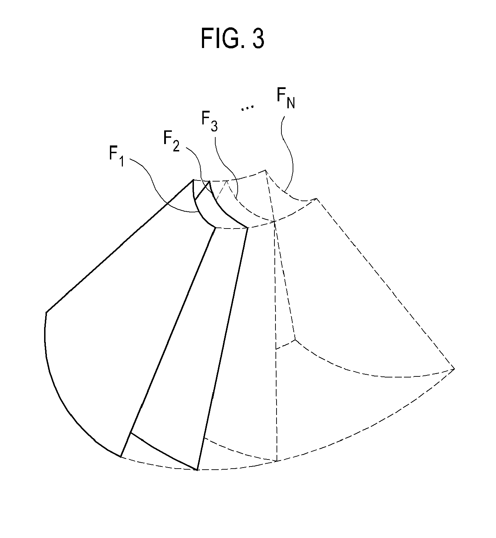

In one embodiment, the Tx signal generating section 220 generates Tx signals for obtaining a plurality of frames Fᵢ (1 ≤ i ≤ N) corresponding to a three-dimensional (3D) ultrasound image at every predetermined time, as shown in FIG. 3.

FIG. 3 is a schematic diagram showing an example of acquiring ultrasound data corresponding to the plurality of frames Fᵢ (1 ≤ i ≤ N). The plurality of frames Fᵢ (1 ≤ i ≤ N) represents sectional planes of the living body (not shown).

Referring back to FIG. 2, the beam former 230 is configured to convert the received signals provided from the ultrasound probe 210 into digital signals. The beam former 230 further applies delays to the digital signals in consideration of the elements and the focal points to output digital receive-focused signals.

The ultrasound data forming section 240 is configured to form ultrasound data corresponding to the frames Fᵢ (1 ≤ i ≤ N) based on the digital receive-focused signals provided from the beam former 230. The ultrasound data forming section 240 further performs various signal processing (e.g., gain adjustment) upon the digital receive-focused signals.

Referring back to FIG 1, the ultrasound system 100 further includes a user input unit 120. The user input unit 120 is configured to receive input information for setting at least three points on the three-dimensional (3D) ultrasound image. The user input unit 120 includes a control panel, a mouse or a keyboard. However, it should be noted herein that the user input unit 120 may not be limited thereto.

The ultrasound system 100 further includes a processing unit 130 in communication with the ultrasound data acquisition unit 110 and the user input unit 120. The processing unit 130 includes a central processing unit, a microprocessor or a graphic processing unit. However, it should be noted herein that the processing unit 130 may not be limited thereto.

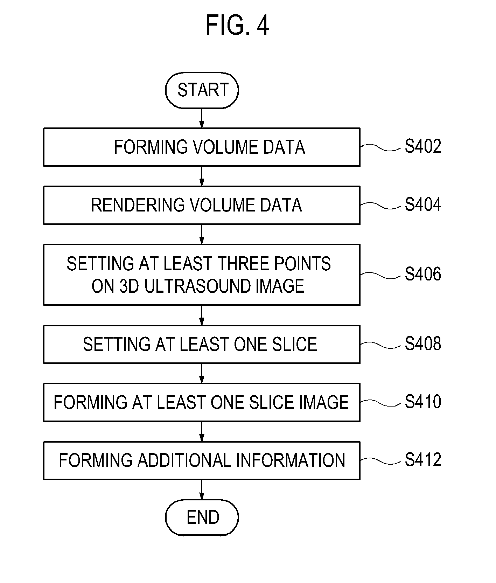

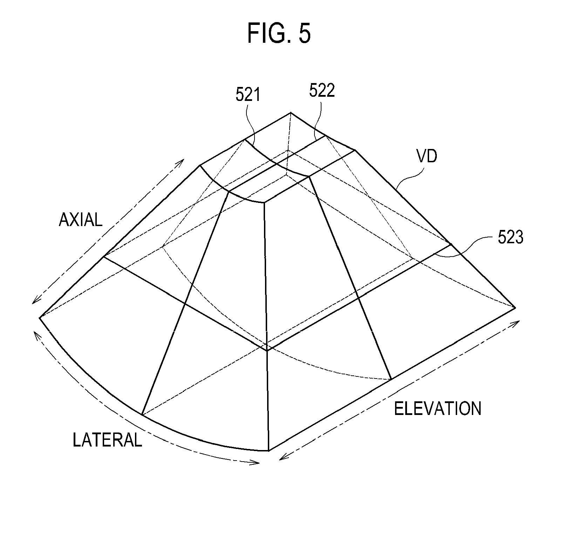

FIG. 4 is a flow chart showing a process of providing slice images with additional information. The processing unit 130 is configured to synthesize the ultrasound data corresponding to the plurality of frames Fᵢ (1 ≤ i ≤ N) to form volume data VD as shown in FIG. 5, at step S402 in FIG. 4.

FIG. 5 is a schematic diagram showing an example of the volume data VD. The volume data VD include a plurality of voxels (not shown) having brightness values. In FIG. 5, reference numerals 521, 522 and 523 represent an A plane, a B plane and a C plane, respectively. The A plane 521, the B plane 522 and the C plane 523 are mutually orthogonal. Also, in FIG. 5, the axial direction is a Tx direction of the ultrasound signals, the lateral direction is a longitudinal direction of the elements, and the elevation direction is a swing direction of the elements, i.e., a depth direction of the 3D ultrasound image.

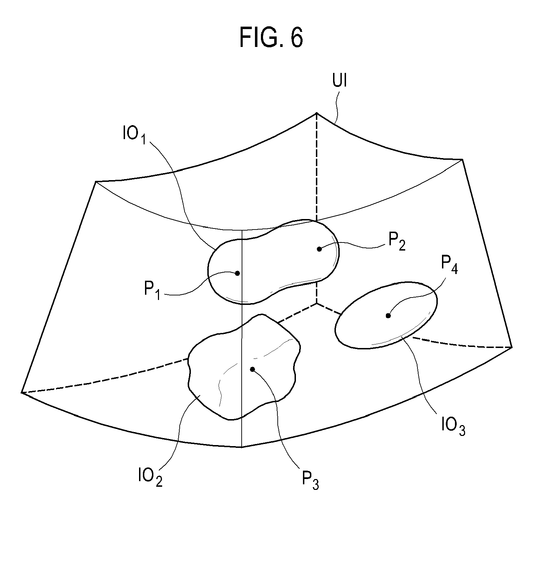

The processing unit 130 is configured to perform a rendering upon the volume data VD to form the 3D ultrasound image UI as shown in FIG. 6, at step S404 in FIG. 4. The 3D ultrasound image UI is displayed on a display unit 150 in FIG. 1. Thus, the user sets the at least three points on the 3D ultrasound image UI displayed on the display unit 150 by using the user input unit 120.

The processing unit 130 is configured to set the at least three points on the 3D ultrasound image based on the input information provided from the user input unit 120, at step S406 in FIG. 4. In one embodiment, the processing unit 130 sets points P₁ to P₄ on corresponding positions of the 3D ultrasound image UI as shown in FIG. 6, based on the input information provided from the user input unit 120. In FIG. 6, reference numerals IO₁ to IO₃ represent the target objects within the living body.

The processing unit 130 is configured to set at least one slice based on the at least three points set on the 3D ultrasound image, at step S408 in FIG. 4.

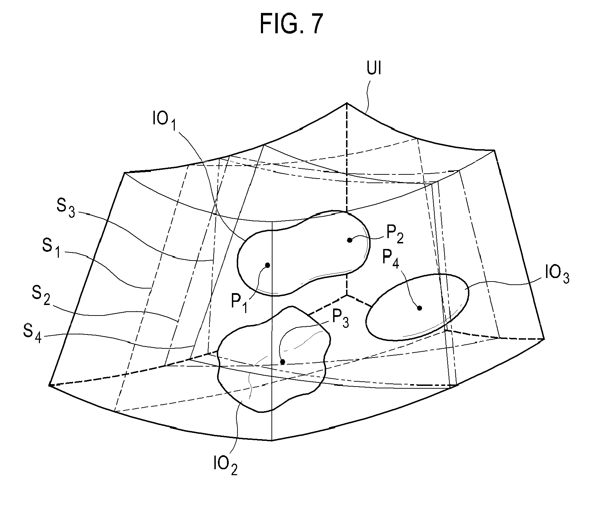

In one embodiment, the processing unit 130 sets point groups from the points P₁ to P₄ that are set on the 3D ultrasound image UI as shown in FIG. 6, wherein each of the point groups includes three points. That is, The processing unit 130 sets: a first point group including points P₁, P₂ and P₃ from the points P₁ to P₄; a second point group including points P₁, P₂ and P₄ from the points P₁ to P₄; a third point group including points P₁, P₃ and P₄ from the points P₁ to P₄; and a fourth point group including points P₂, P₃ and P₄ from the points P₁ to P₄. The processing unit 130 further sets: a first slice S₁ passing through the points P₁, P₂ and P₃ of the first point group; a second slice S₂ passing through the points P₁, P₂ and P₄ of the second point group; a third slice S₃ passing through the points P₁, P₃ and P₄ of the third point group; and a fourth slice S₄ passing through the points P₂, P₃ and P₄ of the fourth point group on the 3D ultrasound image UI as shown in FIG. 7.

The processing unit 130 is configured to form at least one slice image corresponding to the at least one slice in consideration of geometry information of the at least one slice set on the 3D ultrasound image based on the volume data VD, at step S410 in FIG. 4.

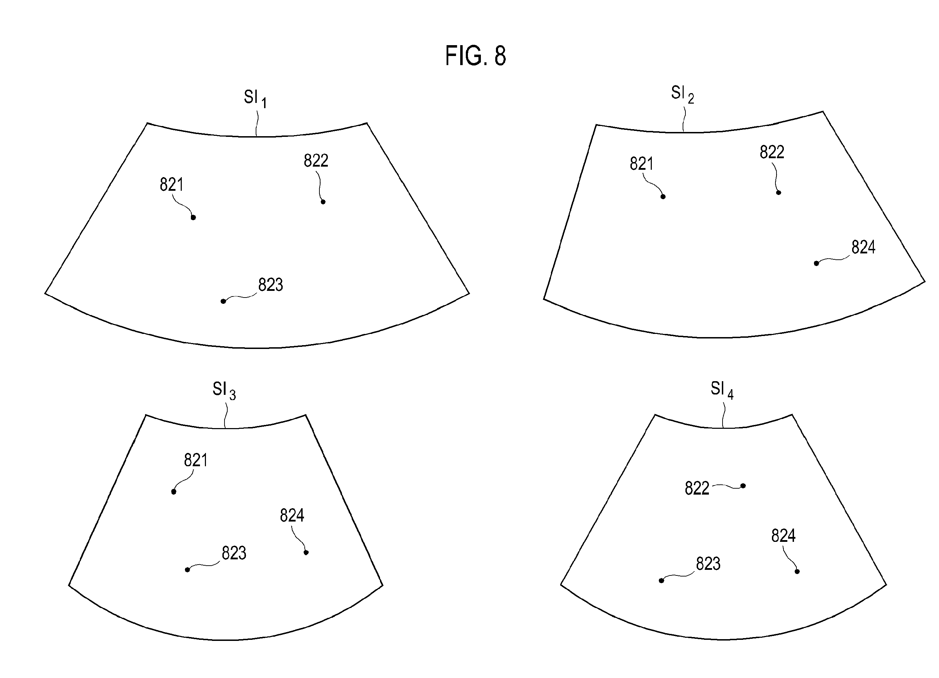

In one embodiment, the processing unit 130 forms a first slice image SI₁ corresponding to the first slice S₁ in consideration of geometry information of the first slice S₁ based on the volume data VD, as shown in FIG. 8. The processing unit 130 further forms a second slice image SI₂ corresponding the second slice S₂ in consideration of geometry information of the second slice S₂ based on the volume data VD, as shown in FIG. 8. The processing unit 130 further forms a third slice image SI₃ corresponding to the third slice S₃ in consideration of geometry information of the third slice S₃ based on the volume data VD, as shown in FIG. 8. The processing unit 130 further forms a fourth slice image SI₄ corresponding to the fourth slice S₄ in consideration of geometry information of the fourth slice S₄ based on the volume data VD, as shown in FIG. 8.

The processing unit 130 is configured to form additional information corresponding to positions of the at least three points, at step S412 in FIG. 4.

In one embodiment, the processing unit 130 forms the additional information representing the at least three points on the at least one slice image corresponding to the at least one slice. For example, the processing unit 130 forms first additional information 821, 822 and 823 representing the points P₁, P₂ and P₃ on the first slice image SI₁, second additional information 821, 822 and 824 representing the points P₁, P₂ and P₄ on the second slice image SI₂, third additional information 821, 823, and 824 representing the points P₁, P₃ and P₄ on the third slice image SI₃, and fourth additional information 822, 823 and 824 representing the points P₂, P₃ and P₄ on the fourth slice image SI₄, based on the input information provided from the user input unit 120 and the geometry information of the first to fourth slices S₁ to S₄.

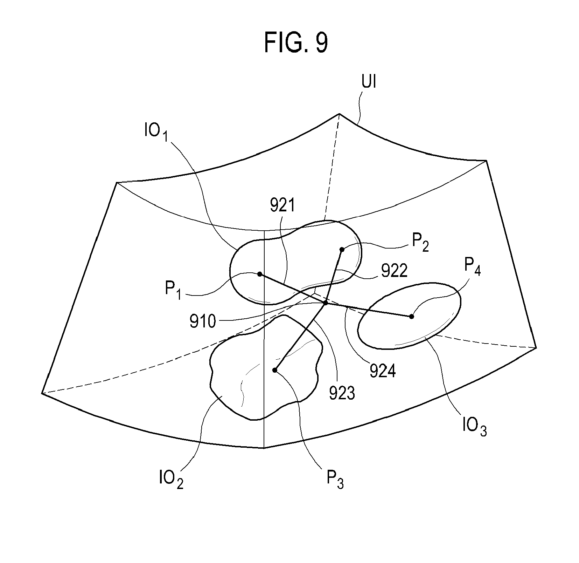

In another embodiment, the processing unit 130 detects a center of the 3D ultrasound image, measure distances between the center and each of the at least three points, and form the additional information representing the distances between the center and each of the at least three points. For example, the processing unit 130 detects the center 910 of the 3D ultrasound image UI, as shown in FIG. 9. The processing unit 130 further measures a first distance between the center 910 and the point P₁, a second distance between the center 910 and the point P₂, a third distance between the center 910 and the point P₃, and a fourth distance between the center 910 and the point P₄. The processing unit 130 further forms first additional information 921 representing the first distance, second additional information 922 representing the second distance, third additional information 923 representing the third distance, and fourth additional information 924 representing the fourth distance. The first to fourth additional information 921 to 924 is represented as at least one of a symbol, a color, a text, a numerical value, a brightness of the color, a diagram and the like.

Referring back to FIG. 1, the ultrasound system 100 further includes a storage unit 140. The storage unit 140 stores the ultrasound data acquired by the ultrasound data acquisition unit 110. The storage unit 140 further stores the volume data formed by the processing unit 130. The storage unit 140 further stores the input information received from the user input unit 120.

The ultrasound system 100 further includes the display unit 150. The display unit 150 displays the 3D ultrasound image formed by the processing unit 130. The display unit 150 further displays the at least one slice image and the additional information formed by the processing unit 130. The display unit 150 includes a cathode ray tube, a liquid crystal display, an organic light emitting diode and the like.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit and receive ultrasound signals to and from a living body to output ultrasound data;
a user input unit configured to receive input information for setting at least three points on a three-dimensional ultrasound image from a user; and
a processing unit in communication with the ultrasound data acquisition unit and the user input unit, the processing unit being configured to form volume data based on the ultrasound data, render the volume data to form the three-dimensional ultrasound image, set the at least three points on the three-dimensional ultrasound image based on the input information, set at least one slice on the three-dimensional ultrasound image based on the at least three points and form at least one slice image corresponding to the at least one slice and additional information corresponding to positions of the at least three points based on the volume data and the input information.

2. The ultrasound system of Claim 1, wherein the processing unit is configured to:
set at least one point group from the at least three points, wherein each of the at least one point group includes three points from the at least three points and the at least one slice passes the at least one point group.

3. The ultrasound system of Claim 1, wherein the processing unit is configured to form the additional information representing the positions of the at least three points on the at least one slice image.

4. The ultrasound system of Claim 1, wherein the processing unit is configured to detect a center of the three-dimensional ultrasound image, measure distances between the center and each of the at least three points, and form the additional information representing the distances.

5. The ultrasound system of Claim 4, wherein the additional information comprises at least one of a symbol, a color, a text, a numerical value, a brightness of the color and a diagram.

6. A method of providing slice images with additional information, comprising:
a) transmitting and receiving ultrasound signals to and from a living body to output ultrasound data;
b) forming volume data based on the ultrasound data;
c) rendering the volume data to form the three-dimensional ultrasound image;
d) receiving input information for setting at least three points on the three-dimensional ultrasound image from a user;
e) setting the at least three points on the three-dimensional ultrasound image based on the input information;
f) setting at least one slice on the three-dimensional ultrasound image based on the at least three points; and
g) forming at least one slice image corresponding to the at least one slice and additional information corresponding to positions of the at least three points.

7. The method of Claim 6, wherein the step f) comprises:
setting at least one point group from the at least three points, wherein each of the at least one point group includes three points from the at least three points and the at least one slice passes the at least one point group.

8. The method of Claim 6, wherein the step g) comprises:
forming the additional information representing the positions of the at least three points on the at least one slice image.

9. The method of Claim 6, wherein the step g) comprises:
detecting a center of the three-dimensional ultrasound image;
measuring distances between the center and each of the at least three points; and
forming the additional information representing the distances.

10. The method of Claim 9, wherein the additional information comprises at least one of a symbol, a color, a text, a numerical value, a brightness of the color and a diagram.
